# EUROPEAN PATENT SPECIFICATION

(11) **EP 1 414 355 B1**
(45) Date of publication and mention of the grant of the patent: **28.06.2006**
(21) Application number: 02751364.7
(22) Date of filing: 07.08.2002
(51) Int. Cl.: A61B 17/15

(54) **TIBIAL RESECTION GUIDE**
TIBIALE RESEKTIONSFÜHRUNG
GUIDE DE RESECTION TIBIALE

(30) Priority: 10.08.2001 GB 0119540
(43) Date of publication of application: 06.05.2004
(73) Proprietor: Depuy International Limited, Beeston, Leeds LS11 8DT (GB)
(72) Inventor: KEENE, Greg, c/o Sports Med SA, Stepney, S.A. 5069 (AU)
(74) Representative: Belcher, Simon James
(86) International application number: PCT/GB2002/003542
(87) International publication number: WO 2003/013371

(56) References cited:
- WO-A-01/00096
- US-A- 4 759 350

## Description

This invention relates to a tibial resection guide for preparing a knee joint for implantation of a prosthesis. Such a guide is e.g. known from US-A-4 759 350.

Accurate resection of a patient's bones is crucially important for success in an operation to implant a joint prosthesis. The resection plane must be accurately located: it is generally desirable to minimise the amount of bone that is removed, while being sure to remove all bone tissue that is defective. The orientation of the plane relative to the anatomical axis must also be accurately controlled to ensure alignment of the articulating surfaces of the joint after surgery, throughout the full range of joint motion.

Accurate location of the tibial resection plane in a knee joint is commonly established using an alignment guide which includes an alignment rod which can be fastened to the tibia distally, close to the ankle. The rod extends along the tibial, parallel to the axis of the tibia. The resection plane can then be defined relative to the tibial axis using a cutting block which can be attached to the alignment guide.

In order to be able to control fully the location and orientation of the tibial resection plane, the surgeon should be able to control (a) the height of the cutting block along the tibial axis, (b) the angle of the cutting block about the anterior-posterior axis (lateral slope), and (c) the angle of the cutting block about the medial-lateral axis (posterior slope). The angle of the cutting block about the anterior-posterior axis can be important to correct or to prevent varus-valgus deformities.

The multiple degrees of freedom of movement of the cutting block which defines the tibial resection plane can make it difficult to ensure that the resection plane is identified with optimal accuracy.

The present invention provides a tibial resection guide in which the alignment rod has a proximal slot formed in it extending substantially parallel to the alignment rod axis, which is a sliding fit on a guide pin.

Accordingly, in one aspect, the invention provides a tibial resection guide for use in preparing a knee joint for implantation of a prosthesis, which comprises:
a. an alignment rod which can be positioned against the tibia, which can be fastened to the tibia towards its distal end, and which has a proximal slot formed in it extending substantially parallel to the axis of the alignment rod,
b. a cutting block at the proximal end of the alignment rod having a saw guide slot in it to guide a saw during resection of the patient's tibia,
c. a guide pin which can be fastened into the tibia, the slot in the alignment rod being a sliding fit over the guide pin, and
the cutting block or the alignment rod towards its proximal end having fixation holes for receiving fixation pins, to fix the cutting block and the alignment rod against further movement relative to the guide pin.

The resection guide of the present invention has the advantage that it allows the cutting block to be located relative to the tibial axis in an initial location step. Subsequent movement of the block to select (a) the appropriate height of the cutting block along the tibial axis, (b) the appropriate angle of the cutting block about the anterior-posterior axis (lateral slope), and (c) the appropriate angle of the cutting block about the medial-lateral axis (posterior slope) can be performed by a combination of sliding and pivoting the alignment rod relative to the guide pin. This can facilitate accurate location of the alignment rod and cutting block prior to resection of the tibia.

Preferably, the alignment rod is constructed so that its length can be adjusted after it has been fastened to the tibia at or towards its distal end. This allows the height of the cutting block along the tibial axis to be selected after the alignment rod has been fastened to the tibia at the distal end. For example, the alignment rod can be constructed as two telescoping sections (preferably with the two parts having cooperating dovetail cross-sections, or optionally with one of the sections being hollow and receiving the end of the other section within it), in which a screw threaded clamp is used to lock the sections against movement when the alignment rod has a desired length.

The guide can include a distal fixation bracket at or towards distal end of the alignment rod, which can engage the patient's leg to fasten the alignment rod relative to the tibia. Suitable brackets for fastening a tibial alignment rod distally are known, for example as sold by DePuy Orthopaedics Inc for use with their LCS^{™} knee prosthesis system. Such brackets can be fastened to the patient's leg by one or more of a clamp which extends around the leg, and bone screws.

Preferably, the bracket includes means for adjusting the location of the distal end of the alignment rod relative to the bracket. This allows the position of the alignment rod to be moved relative to the tibia after the bracket has been fastened to the patient's leg. Preferably, the bracket includes a member which extends generally perpendicularly to the alignment rod, and which provides a track on which the alignment rod can slide, to provide movement along a pre-determined path of the distal end of the alignment rod relative to the bracket. For example, the member can be arranged to extend parallel to the anterior/posterior axis. Accordingly, movement of the end of the alignment rod along the member will give rise to a change in the angle of the cutting block about the medial-lateral axis. This can therefore be used to adjust the posterior slope of the tibial resection plane. Similarly, movement of the end of the alignment rod in a direction parallel to the medial/lateral axis can be used to adjust the lateral slope of the tibial resection plane.

The movement of the alignment rod on the member can be achieved for example by means of an opening extending through the alignment rod which can receive the member in a sliding fit. A screw threaded clamp is used to lock the alignment rod and the member against movement when the alignment rod is in a desired location. When the member extends generally parallel to the anterior/posterior axis, the member can be arranged to extend towards the patient's second toe.

The guide can include a stylus component which can be fitted to the alignment rod at about its proximal end. The stylus component can be used to determine the position of the cutting block along the tibial axis relative to the proximal end of the tibia. The distance from the tibial bearing surface and the resection plane will be predetermined according to the configuration of the tibial component of the knee prosthesis which is to be implanted.

The stylus component can be fixed to the alignment rod or to the cutting block (when it is separable from the alignment rod).

The cutting block is preferably separable from the alignment rod. This has the advantage that, once the cutting block has been fixed in a desired location on the tibia, the alignment rod can be removed. However, for some applications and with a view to minimising inventory, it might be preferred for the cutting block and the alignment rod to be formed as one part.

Especially when the cutting block is separable from the alignment rod, it will be preferred for the fixation holes to be provided in the cutting block.

The cutting block can have guide surfaces for more than one cut. Generally, the cutting block will have a transverse slot which is used to define the location of the transverse resection of the tibia. When the cutting block is used to prepare a knee joint for implantation of a prosthesis in just one of the compartments, the cutting block can also define a generally vertical cutting plane. The vertical cutting plane will intersect the transverse cutting plane, allowing a portion of the tibia, including the bearing surface which contacts just one of the femoral condyles, to be removed. The vertical cutting plane can be defined by a slot, or by an upstanding abutment whose side surface provides the guide surface.

When the resection guide is being used to prepare a knee joint for implantation of a prosthesis in just one of the compartments, the cutting block can extend primarily to just one side of the alignment rod, according to whether the compartment in which the prosthesis is to be implanted is medial or lateral.

Preferably, the slot in the alignment rod in which the guide pin is received is at least 5 mm deep (measured parallel to the direction in which the guide pin extends), for example at least about 10 mm.

Preferably, the difference between the width of the slot and the transverse dimension of the guide pin (which will be the diameter of the guide pin when it has a circular cross-section) is not more than about 2 mm, more preferably not more than about 1 mm.

If the engagement of the guide pin in the slot in the alignment rod is to allow changes in the angle of the cutting block about the anterior-posterior axis (and hence the lateral slope of the cutting block), it might be necessary for the sidewalls of the slot to be bevelled, or for there to be a greater clearance between the guide pin and the sidewalls of the slot than would otherwise by the case.

Embodiments of the present invention will now be described by way of example with reference to the accompanying drawings, in which:
Figure 1 is an isometric view of the tibial alignment rod of the present invention.
Figure 2 is an isometric view of the top of the alignment rod with a stylus mounted on the cutting block.
Figure 3 is an isometric view of the top of the tibia after resection, showing vertical and transverse resection planes.

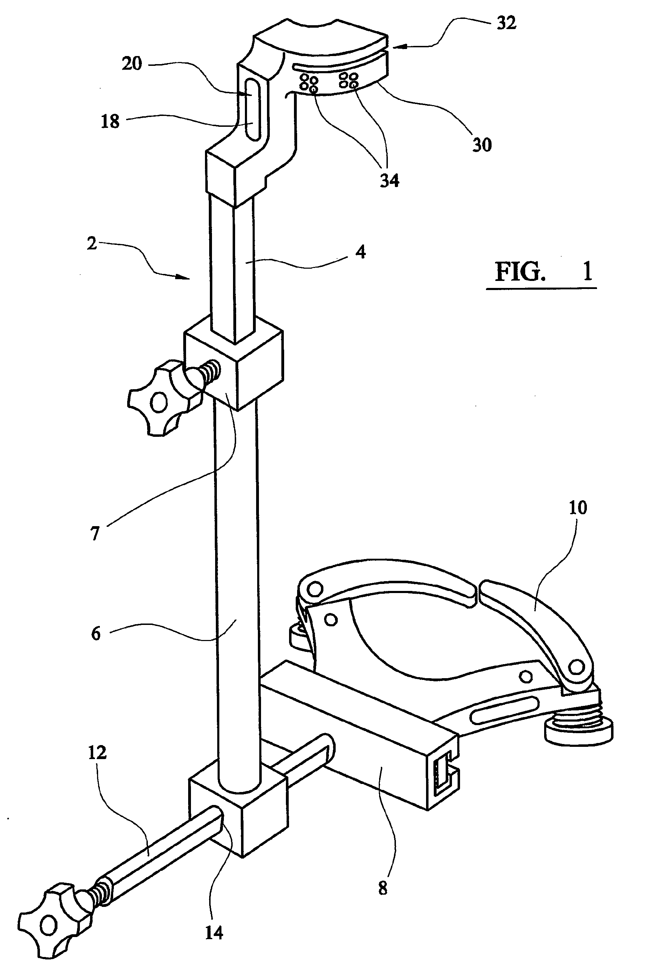

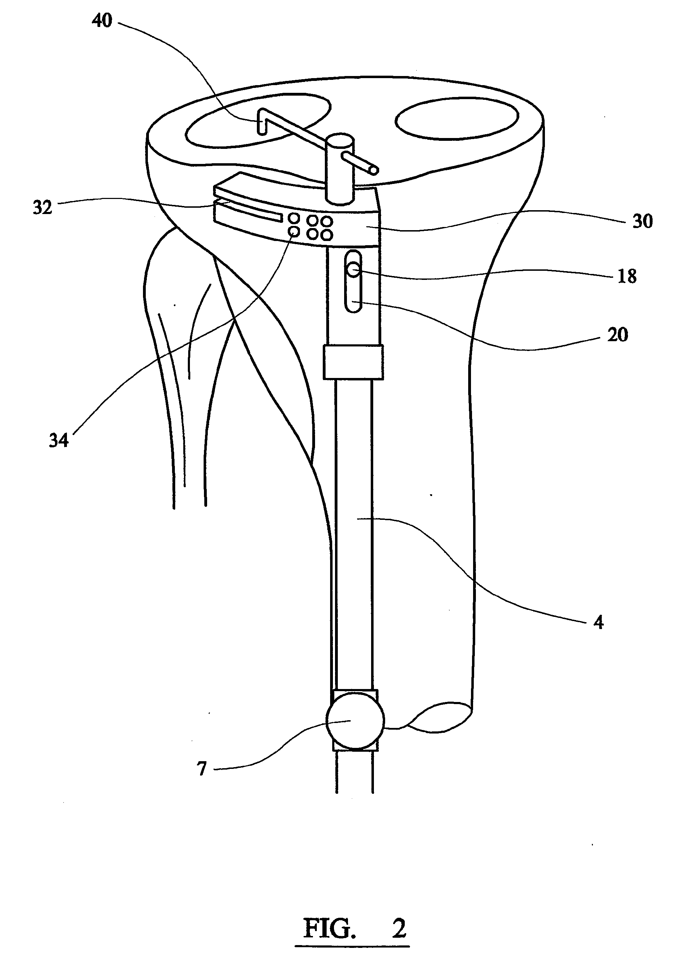

Referring to the drawings, Figures 1 and 2 show a resection guide which includes an alignment rod 2 which can be used to locate a cutting block accurately on a tibia, which can then be used to locate and orientate the resection plane of the tibia, prior to implantation of a knee joint prosthesis.

The alignment rod 2 has upper and lower parts 4, 6 which engage one another telescopically so that the upper part can be slid relative to the lower part to change the overall length of the alignment rod. The parts of the alignment rod can be locked against further movement by means of a screw-threaded clamp 7 which is received in a threaded opening in one of the parts, and acts against the other part.

The alignment rod has a cutting block 30 at its upper (proximal) end. The cutting block has a transverse saw guide slot 32 in it to guide a saw during resection of the patient's tibia.

The cutting block is fixed permanently at the top of the alignment rod, for example as a result of being formed integrally by casting with the alignment rod end cap (having the vertical slot formed in it). The cutting block has fixation holes 34 by which the position of the cutting block relative to the tibia can be fixed.

The resection guide includes a bracket 8 for fastening the alignment rod at its distal end to the patient's leg. The bracket extends around the patient's ankle and can be tightened against the ankle to prevent movement, either along or around the leg. The collar portion 10 of the bracket which extends around the ankle can be appropriately configured to engage anatomical features of the leg so that rotation of the bracket around the leg is prevented. Fixation pins can be used if necessary to prevent unwanted movement.

The bracket includes an elongate member 12 which extends from the collar portion 10 in a direction which is parallel to the anterior/posterior axis. The member will generally extend towards the patient's second toe.

The alignment rod has connection block 14 at its distal end which has a hole in it which can receive the elongate member 12 in a sliding fit. Accordingly, when the bracket 8 has been fastened to the patient's leg, the distal end of the alignment rod can be moved relative to the leg, along the anterior/posterior axis. The elongate member can be locked against movement in a desired position relative to the alignment rod, for example by means of a screw-threaded clamp which can be received in a threaded opening in the connection block and acts against the member.

At its upper end, the alignment rod has a slot 18 formed in it, extending parallel to the axis of the rod. The length of the slot is about 30 mm and its width is about 7 mm. The resection guide includes a guide pin 20 which is a sliding fit in the slot. The pin can be drilled percutaneously into the tibia through the vertical slot 18 to stabilise the alignment rod. The pin will generally be drilled into the tibia, approximately centrally along the medial/lateral axis, with the alignment rod aligned to the anterior border of the tibia or to the tibial eminence. The combination of the bracket at the distal end and the pin in the slot at the proximal end serves to stabilise the alignment rod relative to the tibia while final adjustments are made to the position of the cutting block.

A stylus 40 can be fitted to the cutting block as shown in Figure 2. The stylus can extend into the region in the joint between the articulating surfaces to contact a predetermined point on the tibia. That point will generally be on the bearing surface. It allows the cutting block to be positioned accurately relative to the bearing surface with the saw guide slot 32 a predetermined distance from the bearing surface. This adjustment can be made using the screw-threaded clamp 7 to allow relative movement of the upper and lower alignment rod parts 4, 6.

The posterior slope of the transverse tibial resection plane can be adjusted using the screw-threaded clamp 16 to allow movement of the lower end of the alignment rod along the member 12 on the collar portion of the bracket 8.

When the desired position of the cutting block is attained, it is fixed in place relative to the tibia using fixation pins 42 inserted through the fixation holes 34 in the cutting block. Once it has been fixed in this way, it can be used to guide the transverse tibial resection cut, using a saw blade which is positioned in the saw guide slot 32. When a joint prosthesis is to be implanted in just one of the compartments of the knee joint, a vertical saw cut is made, and the position of the cut can be controlled using an upstand on the cutting block (not shown) in the conventional manner.

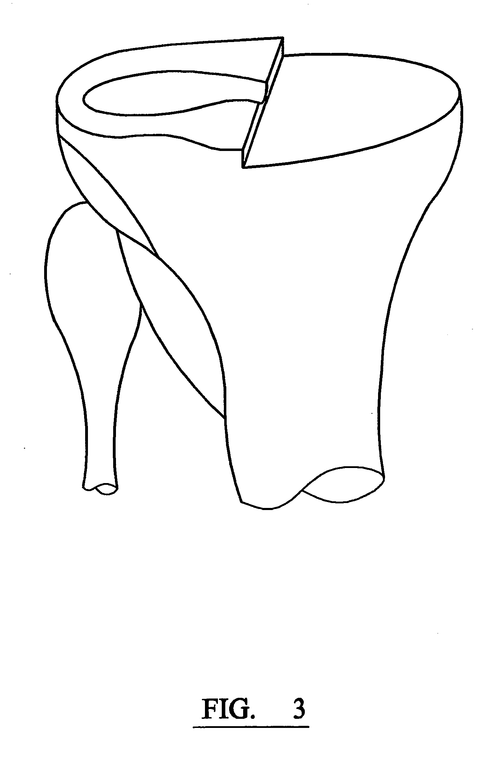

Figure 3 shows the proximal portion of a tibia which has been resected to allow implantation of the tibial component of a uni-compartmental knee prosthesis.

## Claims

1. A tibial resection guide for use in preparing a knee joint for implantation of a prosthesis, which comprises:
a. an alignment rod (2) which can be positioned against the tibia, which can be fastened to the tibia towards its distal end, and which has a proximal slot (18) formed in it extending substantially parallel to the axis of the alignment rod (2);
b. a cutting block (30) at the proximal end of the alignment rod (2) having a saw guide slot (32) in it to guide a saw during resection of the patient's tibia,
c. a guide pin (20) which can be fastened into the tibia, the slot (18) in the alignment rod (2) being a sliding fit over the guide pin (20), and
the cutting block (30) or the alignment rod (2) towards its proximal end having fixation holes (34) for receiving fixation pins, to fix the cutting block (30) and the alignment rod (2) against further movement relative to the guide pin (20).

2. A guide as claimed in claim 1, in which the alignment rod (2) is constructed so that its length can be adjusted after it has been fastened to the tibia at or towards its distal end.

3. A guide as claimed in claim 1, which includes distal fixation bracket (10) at or towards distal end of the alignment rod (2), which can engage the patient's leg to fasten the alignment rod (2) relative to the tibia.

4. A guide as claimed in claim 3, which includes means for adjusting the location of the distal end of the alignment rod (2) relative to the bracket (10).

5. A guide as claimed in claim 3, in which the bracket (10) includes a member which extends generally perpendicularly to the alignment rod, and which provides a track on which the alignment rod (2) can slide, to provide movement along a pre-determined path of the distal end of the alignment rod (2) relative to the bracket (10).

6. A guide as claimed in claim 1, which includes a stylus (40) component which can be fitted to the alignment rod (2) at about its proximal end.

7. A guide as claimed in claim 1, in which the fixation holes (34) are provided in the cutting block (30).

## Patentansprüche

1. Tibiale Resektionsführung zur Verwendung bei der Vorbereitung eines Kniegelenkes zur Implantation einer Prothese, mit:
a) einem Einstellstab (2), welcher an der Tibia angeordnet werden kann, an der Tibia zu seinem distalen Ende hin befestigt werden kann und einen hierin gebildeten proximalen Schlitz (18) aufweist, welcher sich im wesentlichen parallel zur Achse des Einstellstabes (2) erstreckt;
b) einem Schneideblock (30) am proximalen Ende des Einstellstabes (2) mit einem Sägeführungsschlitz (32), um während einer Resektion der Tibia eines Patienten eine Säge zu führen; und
c) einem Führungsstift (20), welcher in der Tibia befestigt werden kann, wobei der Schlitz (18) im Einstellstab (2) einen Gleitsitz über den Führungsstift (20) bildet und wobei der Schneideblock (30) oder der Einstellstab (2) zu seinem proximalen Ende hin Fixierungslöcher (34) zum Aufnehmen von Fixierungsstiften aufweist, um den Schneideblock (30) und den Einstellstab (2) gegen eine weitere Bewegung relativ zum Führungsstift (20) zu fixieren.

2. Tibiale Resektionsführung nach Anspruch 1, **dadurch gekennzeichnet, daß** der Einstellstab (2) so konstruiert ist, daß seine Länge eingestellt werden kann, nachdem er an seinem distalen Ende oder zu seinem distalen Ende hin an der Tibia befestigt ist.

3. Tibiale Resektionsführung nach Anspruch 1, **gekennzeichnet durch** eine distale Fixierungsklammer (10) am distalen Ende des Einstellstabes (2) oder zum distalen Ende des Einstellstabes (2) hin, die das Bein des Patienten erfassen kann, um den Einstellstab (2) in Bezug auf die Tibia zu befestigen.

4. Tibiale Resektionsführung nach Anspruch 3, **gekennzeichnet** duch Mittel zum Einstellen des Ortes des distalen Endes des Einstellstabes (2) in Bezug auf die zur Klammer (10).

5. Tibiale Resektionsführung nach Anspruch 3, **dadurch gekennzeichnet, daß** die Klammer (10) ein Mittel umfaßt, welches sich im wesentlichen senkrecht zum Einstellstab erstreckt und eine Bahn bereitstellt, auf welcher der Einstellstab (2) gleiten kann, um für das distale Ende des Einstellstabes (2) im Bezug auf die Klammer (10) eine Bewegung entlang eines vorbestimmten Pfades vorzusehen.

6. Tibiale Resektionsführung nach Anspruch 1, **gekennzeichnet durch** eine Stiftkomponente (40), welche am Einstellstab (2) an seinem proximalen Ende angepaßt werden kann.

7. Tibiale Resektionsführung nach Anspruch 1, **dadurch gekennzeichnet, daß** die Fixierungslöcher (34) im Schneideblock (30) vorgesehen sind.

## Revendications

1. Guide de résection tibiale pour utilisation dans la préparation d'une articulation du genou pour l'implantation d'une prothèse, qui comprend :
a. une tige d'alignement (2) qui peut être positionnée contre le tibia, qui peut être fixée sur le tibia vers son extrémité distale, et qui a une fente proximale (18) formée dans celle-ci s'étendant sensiblement parallèlement à l'axe de la tige d'alignement (2),
b. un bloc de coupe (30) à l'extrémité proximale de la tige d'alignement (2) ayant une fente de guidage de scie (32) dans celui-ci pour guider une scie pendant la résection du tibia du patient,
c. une broche de guidage (20) qui peut être fixée dans le tibia, la fente (18) dans la tige d'alignement (2) étant un emmanchement glissant sur la broche de guidage (20), et
le bloc de coupe (30) ou la tige d'alignement (2) vers son extrémité proximale ayant des trous de fixation (34) pour recevoir des broches de fixation, pour empêcher le bloc de coupe (30) et la tige d'alignement (2) de bouger par rapport à la broche de guidage (20).

2. Guide selon la revendication 1, dans lequel la tige d'alignement (2) est construite de façon à ce que sa longueur puisse être ajustée après qu'elle a été fixée sur le tibia à ou vers son extrémité distale.

3. Guide selon la revendication 1, qui comprend un étrier de fixation distal (10) à ou vers l'extrémité distale de la tige d'alignement (2), qui peut mettre en prise la jambe du patient pour fixer la tige d'alignement (2) par rapport au tibia.

4. Guide selon la revendication 3, qui comprend un moyen pour régler la position de l'extrémité distale de la tige d'alignement (2) par rapport à l'étrier (10).

5. Guide selon la revendication 3, dans lequel l'étrier (10) comprend un élément qui s'étend globalement perpendiculairement à la tige d'alignement, et qui fournit un rail sur lequel la tige d'alignement (2) peut glisser, pour permettre un mouvement le long d'un chemin prédéterminé de l'extrémité distale de la tige d'alignement (2) par rapport à l'étrier (10).

6. Guide selon la revendication 1, qui comprend un composant de stylet (40) qui peut être monté sur la tige d'alignement (2) près de son extrémité proximale.

7. Guide selon la revendication 1, dans lequel les trous de fixation (34) sont prévus dans le bloc de coupe (30) .
